# EUROPEAN PATENT SPECIFICATION

(11) **EP 4 016 162 B1**
(45) Date of publication and mention of the grant of the patent: **25.06.2025**
(21) Application number: 20851701.1
(22) Date of filing: 04.08.2020
(51) Int. Cl.: G02B 23/24, A61B 1/00, A61B 1/045, A61B 1/06, A61B 5/107

(54) **ENDOSCOPE SYSTEM AND METHOD FOR OPERATING SAME**
ENDOSKOPISCHES SYSTEM UND VERFAHREN ZUM BETRIEB DAVON
SYSTÈME D'ENDOSCOPE ET SON PROCÉDÉ DE FONCTIONNEMENT

(30) Priority: 13.08.2019 JP 2019148607; 23.03.2020 JP 2020051379
(43) Date of publication of application: 22.06.2022
(73) Proprietor: FUJIFILM Corporation, Tokyo 106-8620 (JP)
(72) Inventor: UTSUNOMIYA, Daisuke, Ashigarakami-gun, Kanagawa 258-8538 (JP)
(74) Representative: Dehns Germany Partnerschaft mbB
(86) International application number: PCT/JP2020/029827
(87) International publication number: WO 2021/029277

(56) References cited:
- WO-A1-2018/055933
- WO-A1-2018/180250
- WO-A1-2019/017018
- JP-A- 2008 125 996
- JP-A- 2020 014 808
- JP-B2- 5 371 366
- US-A1- 2019 167 086
- US-B2- 10 134 185

## Description

### BACKGROUND OF THE INVENTION

### 1. Field of the Invention

The present invention relates to an endoscope system that displays a measurement marker to be used to measure the size of a subject and a method of operating the endoscope system.

### 2. Description of the Related Art

A distance to a subject, the size of a subject, and the like are acquired in an endoscope system that includes a light source device, an endoscope, and a processor device. For example, in WO2018/051680A, a subject is irradiated with illumination light and measurement light and a measurement light-irradiation region, such as spotlight, appears on the subject due to irradiation with beam light. Then, a measurement marker used to measure the size of the subject is displayed in a subject image to correspond to the position of the spotlight.

### SUMMARY OF THE INVENTION

As a method of correctly detecting the position of spotlight from a subject image, there is a color separation method using the color/brightness/chroma saturation and the like of an image, such as RGB/HSV. However, it may be difficult to detect the position of the spotlight in a case where scattered reflection in a living body, residues, or altered (discolored) tissue is included in a subject. Further, a medical doctor as a user may change setting conditions related to the light source device, the endoscope, and the processor device to a color or illumination, which allows a lesion area to be easily detected, in order to facilitate the detection of a lesion. In a case where the setting conditions are changed in this way, the position of the spotlight may not be detected. Accordingly, it is required to set the setting conditions, which are related to the light source device, the endoscope, and the processor device, to a state where the position of the measurement light-irradiation region, such as the spotlight, can be detected and to allow a measurement marker, which corresponds to an observation distance, to be displayed.

An object of the invention is to provide an endoscope system that can set setting conditions related to a light source device, an endoscope, and a processor device to a state where the position of a measurement light-irradiation region formed on a subject by measurement light can be detected and the like in a case where a measurement marker is displayed in an image using measurement light, and a method of operating the endoscope system.

An endoscope system according to an aspect of the invention is as defined in claim 1.

It is preferable that the prohibition setting condition includes a first prohibition setting condition that causes detection of a position of a measurement light-irradiation region, which appears on the subject due to irradiation with the measurement light, from the subject image to be hindered. It is preferable that the subject image includes a measurement light image based on a color of the measurement light, the setting condition includes a special observation mode in which the measurement light image is not used for display of the measurement image, and the first prohibition setting condition includes the special observation mode. It is preferable that the processor performs a control to cancel the special observation mode and to switch a mode to the length measurement mode instead of prohibiting switching of a mode to the length measurement mode in a case where an operation for switching a mode to the length measurement mode is performed in the special observation mode. It is preferable that the measurement light image is a red image.

It is preferable that the prohibition setting condition includes a second prohibition setting condition that causes display of a measurement marker corresponding to an observation distance in the measurement image to be hindered. It is preferable that the second prohibition setting condition includes a use of a zoom function that enlarges or reduces the subject in the subject image.

It is preferable that the processor notifies that the switching of a mode to the length measurement mode is prohibited in a case where the first control for prohibiting the switching of a mode to the length measurement mode is performed. It is preferable that the processor notifies that the setting change operation is disabled in a case where the second control for disabling the setting change operation is performed. It is preferable that the processor notifies that a mode is switched to another mode in a case where the third control for switching a mode to another mode from the length measurement mode is performed.

It is preferable that the measurement marker includes a first measurement marker showing an actual size of the subject or a second measurement marker consisting of an intersection line formed on the subject by the measurement light and gradations formed on the intersection line and serving as an index of the size of the subject.

According to another aspect of the invention, there is provided a method of operating the endoscope system as defined by claim 12.

According to the invention, it is possible to set setting conditions, which are related to a light source device, an endoscope, and a processor device, to a state where the position of a measurement light-irradiation region formed on a subject by measurement light can be detected and to allow a measurement marker, which corresponds to an observation distance, to be displayed in a case where a measurement marker is displayed in an image using measurement light.

### BRIEF DESCRIPTION OF THE DRAWINGS

Fig. 1 is a diagram showing the appearance of an endoscope system.
Fig. 2 is a plan view of a distal end part of an endoscope.
Fig. 3 is a block diagram showing the functions of the endoscope system.
   (A) of Fig. 4 is a diagram illustrating a subject image obtained in a state where a digital zoom function is OFF and (B) of Fig. 4 is a diagram illustrating a subject image obtained in a state where a digital zoom function is ON.
Fig. 5 is a block diagram showing a measurement light-emitting unit.
Fig. 6 is a diagram illustrating a spot SP that is formed on a subject by measurement light.
Fig. 7 is a diagram illustrating a relationship between the distal end part of the endoscope and a near end Px, an intermediate vicinity Py, and a far end Pz in a range Rx of an observation distance.
Fig. 8 is a block diagram showing the functions of a signal processing unit.
Fig. 9 is a diagram illustrating a first control.
Fig. 10 is an image diagram of a monitor that is displayed in a case where the first control is performed.
Fig. 11 is a diagram illustrating a control for canceling a special observation mode and switching a mode to a length measurement mode.
Fig. 12 is a diagram illustrating a second control.
Fig. 13 is an image diagram of the monitor that is displayed in a case where the second control is performed.
Fig. 14 is a diagram illustrating a third control.
Fig. 15 is an image diagram of the monitor that is displayed in a case where the third control is performed.
Fig. 16 is an image diagram showing a spot and a first measurement marker in a case where an observation distance corresponds to the near end Px.
Fig. 17 is an image diagram showing a spot and a first measurement marker in a case where an observation distance corresponds to the intermediate vicinity Py.
Fig. 18 is an image diagram showing a spot and a first measurement marker in a case where an observation distance corresponds to the far end Pz.
Fig. 19 is a diagram illustrating first measurement markers having a cruciform shape, a cruciform shape with gradations, a distorted cruciform shape, a circular-and-cruciform shape, and the shape of a measurement point group.
Fig. 20 is an image diagram showing three concentric circular markers having the same color.
Fig. 21 is an image diagram showing three concentric circular markers having different colors.
Fig. 22 is an image diagram showing distorted concentric circular markers.
Fig. 23 is a flowchart showing a series of flows of the invention.
Fig. 24 is a diagram illustrating a light emission pattern in which spotlight is intermittently applied.
Fig. 25 is an image diagram showing an intersection line and gradations.
Fig. 26 is a diagram illustrating a light emission pattern in which line-like measurement light is intermittently applied.
Fig. 27 is a diagram illustrating stripe pattern light ZPL.
Fig. 28 is a diagram illustrating the light emission patterns of stripe pattern light ZPL having a phase X, stripe pattern light ZPL having a phase Y, and stripe pattern light ZPL having a phase Z.
Fig. 29 is a diagram illustrating measurement light LPL having a grid pattern.
Fig. 30 is a diagram illustrating a light emission pattern in which measurement light having a grid pattern is intermittently applied.
Fig. 31 is a diagram illustrating three-dimensional plane light TPL.
Fig. 32 is a diagram illustrating a light emission pattern in which three-dimensional plane light is intermittently applied.

### DESCRIPTION OF THE PREFERRED EMBODIMENTS

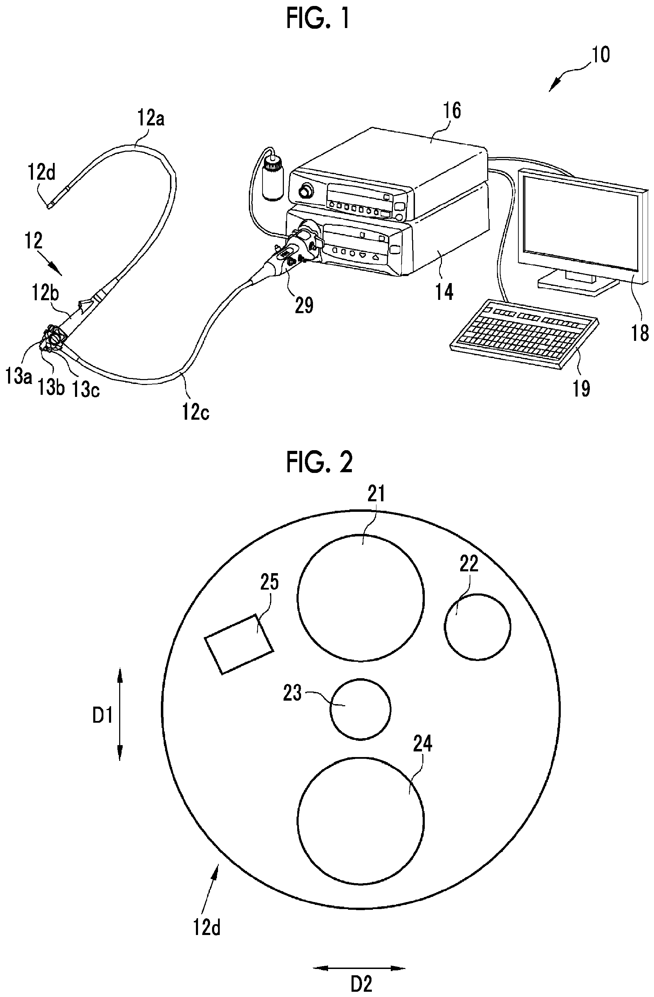

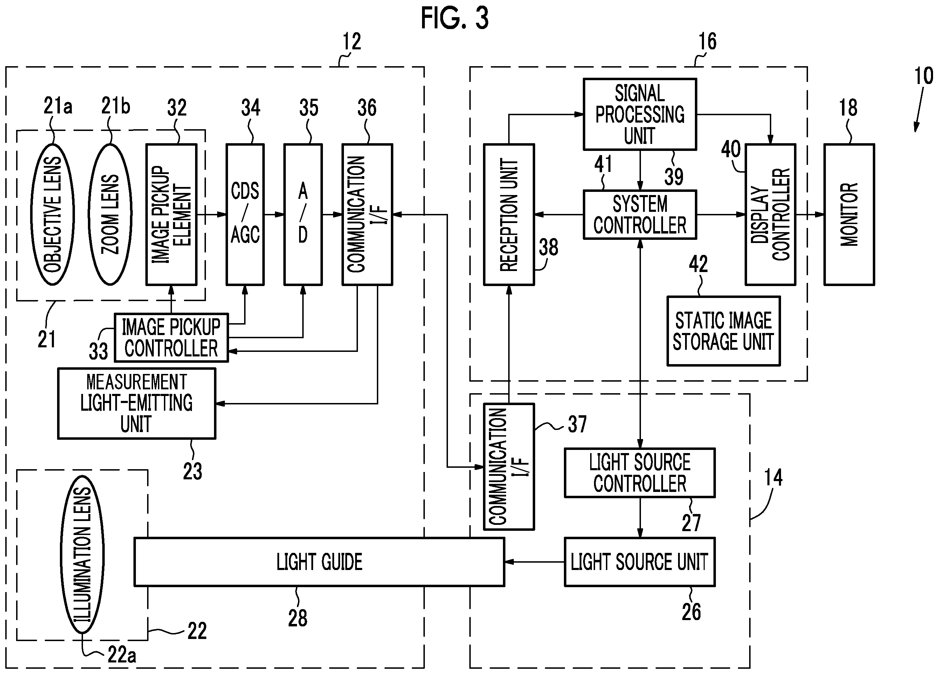

As shown in Fig. 1, an endoscope system 10 includes an endoscope 12, a light source device 14, a processor device 16, a monitor 18 (display), and a user interface 19. The endoscope 12 includes an insertion part 12a that is to be inserted into an object to be examined, an operation part 12b that is provided at the proximal end portion of the insertion part 12a, and a universal cable 12c. The universal cable 12c is a cable in which a light guide 28 (see Fig. 3) for guiding illumination light emitted from the light source device 14, a control line for transmitting control signals used to control the endoscope 12, a signal line for transmitting image signals obtained from the image pickup of an object to be observed, a power line for supplying power to each part of the endoscope 12, and the like are integrated. The distal end of the universal cable 12c is provided with a connector 29 to be connected to the light source device 14.

The light source device 14 generates illumination light by, for example, a semiconductor light source, such as a light emitting diode (LED) or a laser diode (LD), or a halogen lamp, such as a xenon lamp. In a case where the connector 29 is connected to the light source device 14, illumination light is incident on the light guide 28 (see Fig. 3) of the connector 29 and the object to be observed is irradiated with the illumination light from the distal end of the insertion part 12a.

Further, the light source device 14 is electrically connected to the processor device 16 and the connector 29 of the endoscope 12 is connected to the processor device 16 through the light source device 14. The transmission and reception of control signals, image signals, and the like between the light source device 14 and the connector 29 are performed by wireless communication. For this reason, the light source device 14 transmits the control signals and the like, which are wirelessly transmitted to and received from the connector 29, to the processor device 16. Furthermore, the light source device 14 supplies power, which is used to drive the endoscope 12, to the connector 29, and the supply of this power is also wirelessly performed.

The processor device 16 controls the amount and emission timing of illumination light, which is emitted from the light source device 14, and each part of the endoscope 12 and generates an endoscopic image using image signals that are obtained from the image pickup of an object to be observed irradiated with the illumination light. Further, the processor device 16 is electrically connected to the monitor 18 and the user interface 19. The monitor 18 displays the endoscopic image that is generated by the processor device 16, information about the endoscopic image, and the like. The user interface 19 has a function to receive an input operation, such as function settings.

The endoscope 12 has a normal observation mode, a special observation mode, and a length measurement mode, and these three modes are switched by a mode changeover switch 13a that is provided on the operation part 12b of the endoscope 12. The normal observation mode is a mode in which an object to be observed is illuminated with the illumination light. The special observation mode is a mode in which an object to be observed is illuminated with special light different from the illumination light. In the length measurement mode, an object to be observed is illuminated with the illumination light and measurement light and a measurement marker to be used for the measurement of the size and the like of an object to be observed is displayed in a subject image obtained from the image pickup of the object to be observed. The illumination light is light that is used to observe the entire object to be observed by applying brightness to the entire object to be observed. The special light is light that is used to emphasize a specific region of the object to be observed. The measurement light is light that is used for the display of the measurement marker.

Further, the operation part 12b of the endoscope 12 is provided with a freeze switch 13b that is used to give a static image-acquisition instruction to acquire the static image of a subject image. In a case where a user operates the freeze switch 13b, the screen of the monitor 18 is frozen and displayed and an alert sound (for example, "beep") informing the acquisition of a static image is generated together. Then, the static images of the subject image, which are obtained before and after the operation timing of the freeze switch 13b, are stored in a static image storage unit 42 (see Fig. 3) provided in the processor device 16. The static image storage unit 42 is a storage unit, such as a hard disk or a universal serial bus (USB) memory. In a case where the processor device 16 can be connected to a network, the static image of the subject image may be stored in a static image storage server (not shown), which is connected to the network, instead of or in addition to the static image storage unit 42.

A static image-acquisition instruction may be given using an operation device other than the freeze switch 13b. For example, a foot pedal may be connected to the processor device 16, and a static image-acquisition instruction may be given in a case where a user operates the foot pedal (not shown) with a foot. A static image-acquisition instruction may be given by a foot pedal that is used to switch a mode. Further, a gesture recognition unit (not shown), which recognizes the gestures of a user, may be connected to the processor device 16, and a static image-acquisition instruction may be given in a case where the gesture recognition unit recognizes a specific gesture of a user. The gesture recognition unit may also be used to switch a mode.

Further, a sight line input unit (not shown), which is provided close to the monitor 18, may be connected to the processor device 16, and a static image-acquisition instruction may be given in a case where the sight line input unit recognizes that a user's sight line is in a predetermined region of the monitor 18 for a predetermined time or longer. Furthermore, a voice recognition unit (not shown) may be connected to the processor device 16, and a static image-acquisition instruction may be given in a case where the voice recognition unit recognizes a specific voice generated by a user. The voice recognition unit may also be used to switch a mode. Moreover, an operation panel (not shown), such as a touch panel, may be connected to the processor device 16, and a static image-acquisition instruction may be given in a case where a user performs a specific operation on the operation panel. The operation panel may also be used to switch a mode.

As shown in Fig. 2, a distal end part 12d of the endoscope 12 has a substantially circular shape, and is provided with an image pickup optical system 21 that receives light from a subject, an illumination optical system 22 that is used to irradiate the subject with the illumination light, a measurement light-emitting unit 23 that radiates the measurement light to the subject, an opening 24 that allows a treatment tool to protrude toward the subject, and an air/water supply nozzle 25 that is used to supply air and water.

An optical axis Ax of the image pickup optical system 21 extends in a direction perpendicular to the plane of paper. A vertical first direction D1 is orthogonal to the optical axis Ax, and a horizontal second direction D2 is orthogonal to the optical axis Ax and the first direction D1. The image pickup optical system 21 and the measurement light-emitting unit 23 are provided at different positions in the distal end part 12d, and are arranged in the first direction D1.

As shown in Fig. 3, the light source device 14 comprises a light source unit 26 and a light source controller 27. The light source unit 26 generates the illumination light or the special light that is used to illuminate the subject. The illumination light or the special light, which is emitted from the light source unit 26, is incident on the light guide 28, and the subject is irradiated with the illumination light or the special light through an illumination lens 22a. A white light source emitting white light, a plurality of light sources, which include a white light source and a light source emitting another color light (for example, a blue light source emitting blue light), or the like is used as a light source of the illumination light in the light source unit 26. Further, a light source, which emits broadband light including blue narrow-band light used to emphasize superficial information about superficial blood vessels and the like, is used as a light source of the special light in the light source unit 26. The light source controller 27 is connected to a system controller 41 of the processor device 16. Mixed white light, which is a combination of blue light, green light, and red light, may be used as the illumination light. In this case, it is preferable that the illumination optical system 22 is optically designed to allow the irradiation range of green light to be wider than the irradiation range of red light.

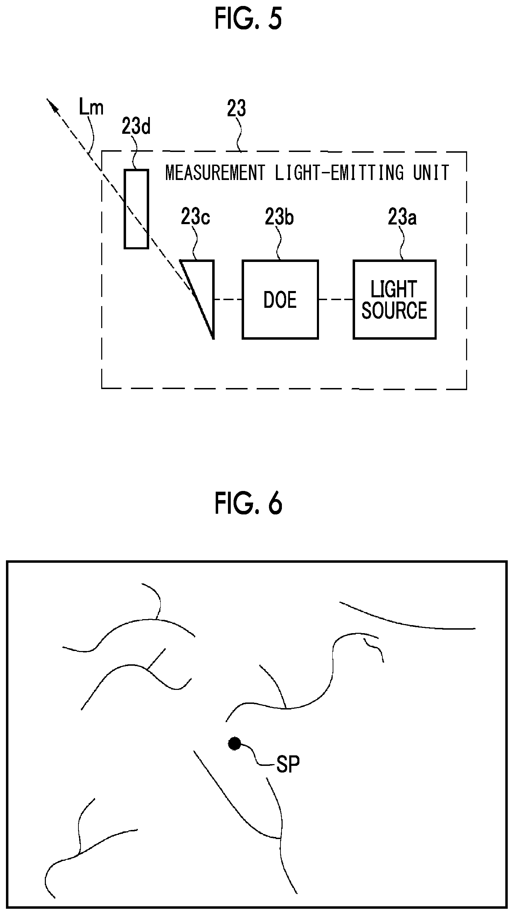

The light source controller 27 controls the light source unit 26 on the basis of an instruction given from the system controller 41. The system controller 41 not only gives an instruction related to light source control to the light source controller 27 but also controls a light source 23a (see Fig. 5) of the measurement light-emitting unit 23. In the case of the normal observation mode, the system controller 41 performs a control to turn on the illumination light and to turn off the measurement light. In the case of the special observation mode, the system controller 41 performs a control to turn on the special light and to turn off the measurement light. In the case of the length measurement mode, the system controller 41 performs a control to turn on the illumination light and to turn on the measurement light.

The illumination optical system 22 includes the illumination lens 22a, and the object to be observed is irradiated with light, which is emitted from the light guide 28, through the illumination lens 22a. The image pickup optical system 21 includes an objective lens 21a, a zoom lens 21b, and an image pickup element 32. Light reflected from the object to be observed is incident on the image pickup element 32 through the objective lens 21a and the zoom lens 21b. Accordingly, the reflected image of the object to be observed is formed on the image pickup element 32.

The zoom lens 21b has an optical zoom function to enlarge or reduce the subject as a zoom function by moving between a telephoto end and a wide end. ON/OFF of the optical zoom function can be switched by a zoom operation part 13c (see Fig. 1) provided on the operation part 12b of the endoscope, and the subject can be enlarged or reduced at a specific magnification ratio in a case where the zoom operation part 13c is further operated in a state where the optical zoom function is ON.

The image pickup element 32 is a color image pickup sensor, and picks up the reflected image of an object to be examined and outputs image signals. It is preferable that the image pickup element 32 is a charge coupled device (CCD) image pickup sensor, a complementary metal-oxide semiconductor (CMOS) image pickup sensor, or the like. The image pickup element 32 used in an embodiment of the invention is a color image pickup sensor that is used to obtain red images, green images, and blue images corresponding to three colors of R (red), G (green), and B (blue). The red image is an image that is output from red pixels provided with red color filters in the image pickup element 32. The green image is an image that is output from green pixels provided with green color filters in the image pickup element 32. The blue image is an image that is output from blue pixels provided with blue color filters in the image pickup element 32. The image pickup element 32 is controlled by an image pickup controller 33.

Image signals output from the image pickup element 32 are transmitted to a CDS/AGC circuit 34. The CDS/AGC circuit 34 performs correlated double sampling (CDS) or auto gain control (AGC) on the image signals that are analog signals. The image signals, which have been transmitted through the CDS/AGC circuit 34, are converted into digital image signals by an analog/digital converter (A/D converter) 35. The digital image signals, which have been subjected to A/D conversion, are input to a communication interface (I/F) 37 of the light source device 14 through a communication interface (I/F) 36.

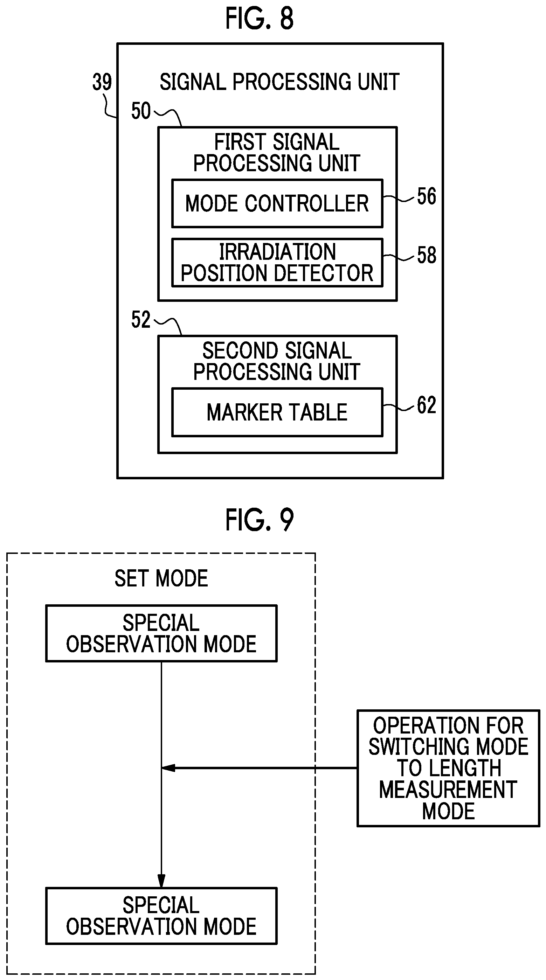

In the processor device 16, programs related to control and the like related to modes, such as a first control, a second control, and a third control to be described later, are incorporated into a program storage memory (not shown). The system controller 41 formed of a processor operates the programs incorporated into the program storage memory, so that the functions of a reception unit 38 connected to the communication interface (I/F) 37 of the light source device 14, a signal processing unit 39, and a display controller 40 are realized. Further, the functions of a first signal processing unit 50, a mode controller 56, an irradiation position detector 58, and a second signal processing unit (see Fig. 8) included in the signal processing unit 39 are also realized by the operation of the programs.

The reception unit 38 receives the image signals, which are transmitted from the communication I/F 37, and transmits the image signals to the signal processing unit 39. A memory, which temporarily stores the image signals received from the reception unit 38, is built in the signal processing unit 39, and the signal processing unit 39 processes an image signal group, which is a set of the image signals stored in the memory, to generate the subject image. The reception unit 38 may directly transmit control signals, which are related to the light source controller 27, to the system controller 41. Further, the processing units (for example, a first signal processing unit 50 and a second signal processing unit 52), which are related to the length measurement mode, of the processor device 16 may be provided in a length measurement processor (not shown) separate from the processor device 16. In this case, the length measurement processor and the processor device 16 are in a state where the length measurement processor and the processor device 16 can communicate with each other so that images or various types of information can be transmitted and received.

In a case where the endoscope 12 is set to the normal observation mode, signal assignment processing for assigning the blue image of the subject image to B channels of the monitor 18, assigning the green image of the subject image to G channels of the monitor 18, and assigning the red image of the subject image to R channels of the monitor 18 is performed in the signal processing unit 39. As a result, a color subject image is displayed on the monitor 18. The same signal assignment processing as that in the normal observation mode is performed even in the length measurement mode. On the other hand, in a case where the endoscope 12 is set to the special observation mode, the red image of the subject image is not used for the display of the monitor 18, the blue image of the subject image is assigned to the B channels and the G channels of the monitor 18, and the green image of the subject image is assigned to the R channels of the monitor 18 in the signal processing unit 39. As a result, a pseudo color subject image is displayed on the monitor 18.

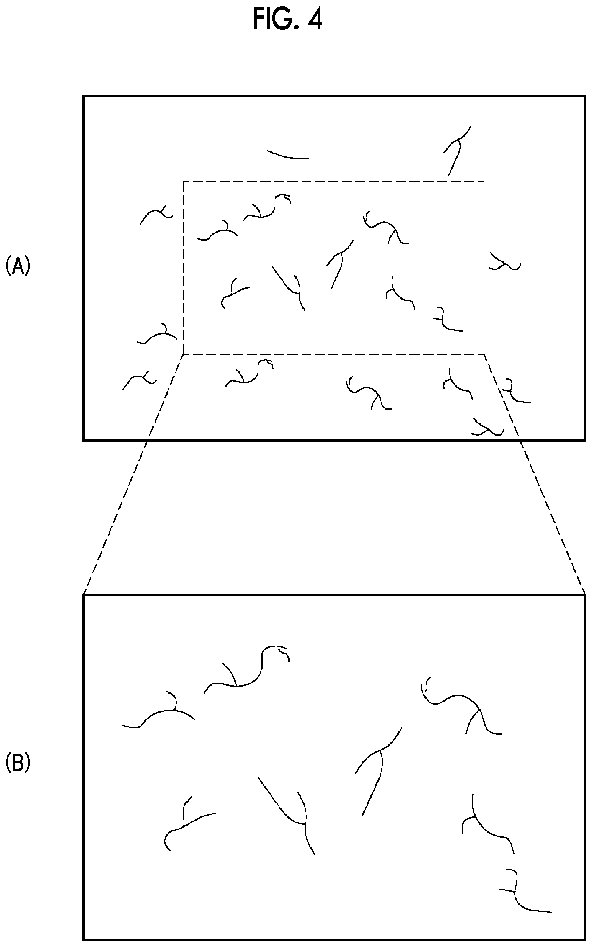

In a case where a digital zoom function is set to ON as a zoom function by the user interface 19, the signal processing unit 39 cuts out a portion of the subject image and enlarges or reduces the cut portion. As a result, the subject is enlarged or reduced at a specific magnification. (A) of Fig. 4 shows a subject image obtained in a state where the digital zoom function is OFF and (B) of Fig. 4 shows a subject image obtained in a state where a central portion of the subject image shown in (A) of Fig. 4 is cut out and enlarged and the digital zoom function is ON. In a case where the digital zoom function is OFF, the enlargement of reduction of the subject using the cutout of the subject image is not performed.

In a case where the endoscope 12 is set to the length measurement mode, the signal processing unit 39 may be adapted to perform structure-emphasis processing for emphasizing structures, such as blood vessels, or color difference-emphasis processing for increasing a color difference between a normal area and a lesion area of the object to be observed on the subject image.

The display controller 40 causes the monitor 18 to display the subject image that is generated by the signal processing unit 39. The system controller 41 performs the control of the image pickup element 32 through the image pickup controller 33 provided in the endoscope 12. The image pickup controller 33 also performs the control of the CDS/AGC circuit 34 and the A/D converter 35 together with the control of the image pickup element 32.

As shown in Fig. 5, the measurement light-emitting unit 23 emits the measurement light obliquely with respect to the optical axis Ax of the image pickup optical system 21. The measurement light-emitting unit 23 comprises a light source 23a, a diffractive optical element (DOE) 23b, a prism 23c, and an emitting section 23d. The light source 23a is to emit light having a color that can be detected by pixels of the image pickup element 32 (specifically visible light), and includes a light emitting element, such as a laser light source (laser diode (LD)) or a light emitting diode (LED), and a condenser lens that condenses light emitted from the light emitting element. The light source 23a is provided on a scope electric board (not shown). The scope electric board is provided in the distal end part 12d of the endoscope, and receives power supplied from the light source device 14 or the processor device 16 and supplies power to the light source 23a.

For example, red (the color of beam light) laser light having a wavelength of 600 nm or more and 650 nm or less is used as the light emitted from the light source 23a in this embodiment, but light having a wavelength in other ranges, for example, green light having a wavelength of 495 nm or more and 570 nm or less may be used. The light source 23a is controlled by the system controller 41, and emits light on the basis of an instruction given from the system controller 41. The DOE 23b converts the light, which is emitted from the light source, into the measurement light that is used to obtain measurement information. It is preferable that the amount of measurement light is adjusted from a standpoint of protecting a human body, eyes, and internal organs and is adjusted to the amount of light enough to cause halation (pixel saturation) in the observation range of the endoscope 12.

The prism 23c is an optical member that is used to change the travel direction of the measurement light converted by the DOE 23b. The prism 23c changes the travel direction of the measurement light so that the measurement light intersects with the visual field of the image pickup optical system 21 including the objective lens 21a. The details of the travel direction of the measurement light will also be described later. A subject is irradiated with measurement light Lm, which is emitted from the prism 23c, through the emitting section 23d that is formed of an optical member.

In a case where the subject is irradiated with the measurement light, a spot SP as a measurement light-irradiation region is formed on the subject as shown in Fig. 6. The position of the spot SP is detected by the irradiation position detector 58 (see Fig. 8), and a measurement marker showing the size of the subject is set according to the position of the spot SP. The set measurement marker is displayed in the subject image. A plurality of types of measurement markers, such as a first measurement marker and a second measurement marker, are included in the measurement marker as described later, and a measurement marker to be displayed in the subject image among the plurality of types of measurement markers can be selected according to a user's instruction. For example, the user interface 19 is used for the user's instruction.

The emitting section 23d may be formed of an auxiliary measurement slit, which is formed at the distal end part 12d of the endoscope, instead of being formed of an optical member. Further, in a case where the emitting section 23d is formed of an optical member, it is preferable that an anti-reflection coating (AR coating) (anti-reflection portion) is provided on the emitting section 23d. The reason why the anti-reflection coating is provided as described above is that it is difficult for the irradiation position detector 58 to be described later to recognize the position of the spot SP formed on the subject by the measurement light in a case where the measurement light is reflected without being transmitted through the emitting section 23d and a ratio of the measurement light with which a subject is irradiated is reduced.

The measurement light-emitting unit 23 has only to be capable of emitting the measurement light to the visual field of the image pickup optical system 21. For example, the light source 23a may be provided in the light source device and light emitted from the light source 23a may be guided to the DOE 23b by optical fibers. Further, the prism 23c may not be used and the directions of the light source 23a and the DOE 23b may be inclined with respect to the optical axis Ax of the image pickup optical system 21 so that the measurement light Lm is emitted in a direction crossing the visual field of the image pickup optical system 21.

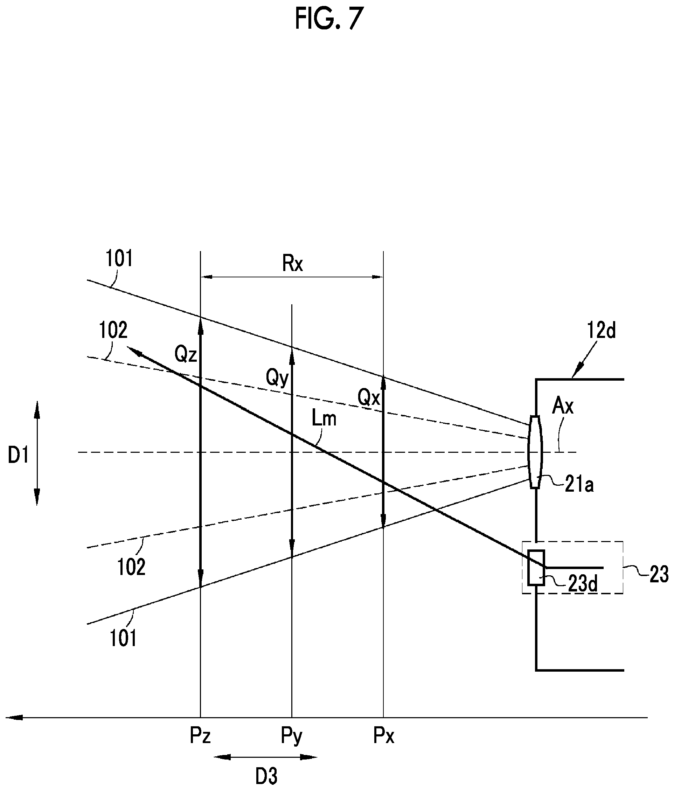

With regard to the travel direction of the measurement light, the measurement light is emitted in a state where the optical axis of the measurement light Lm intersects with the optical axis Ax of the image pickup optical system 21 as shown in Fig. 7. In a case where the subject can be observed in a range Rx of an observation distance, it is understood that the positions (points where the respective arrows Qx, Qy, and Qz intersect with the optical axis Ax) of the spot SP, which is formed on the subject by the measurement light Lm, in image pickup ranges (shown by arrows Qx, Qy, and Qz) at a near end Px, an intermediate vicinity Py, and a far end Pz of the range Rx are different from each other. The image pickup angle of view of the image pickup optical system 21 is represented by a region between two solid lines 101, and measurement is performed in a central region (a region between two dotted lines 102), in which an aberration is small, of this image pickup angle of view.

Since the measurement light Lm is emitted in a state where the optical axis of the measurement light Lm intersects with the optical axis Ax as described above, the size of the subject can be measured from the movement of the position of the spot with respect to a change in observation distance. Then, the image of the subject illuminated with the measurement light is picked up by the image pickup element 32, so that a subject image including the spot SP is obtained. In the subject image, the position of the spot SP varies depending on a relationship between the optical axis Ax of the image pickup optical system 21 and the optical axis of the measurement light Lm and an observation distance. However, the number of pixels showing the same actual size (for example, 5 mm) is increased in the case of a short observation distance, and the number of pixels showing the same actual size is reduced in the case of a long observation distance.

As shown in Fig. 8, the signal processing unit 39 of the processor device 16 controls whether or not the length measurement mode can be executed and the like, and comprises a first signal processing unit 50 that detects the position of the spot SP in the subject image in a state where the execution of the length measurement mode is allowed and a second signal processing unit 52 that sets a measurement marker according to the position of the spot SP. In a case where the endoscope 12 is set to the normal observation mode, the subject image of the subject illuminated with the illumination light is input to the signal processing unit 39. In a case where the endoscope 12 is set to the special observation mode, the subject image of the subject illuminated with the special light is input to the signal processing unit 39. In a case where the endoscope 12 is set to the length measurement mode, the subject image of the subject illuminated with the illumination light and the measurement light is input to the signal processing unit 39.

The first signal processing unit 50 comprises a mode controller 56 that performs a control related to whether or not the length measurement mode can be executed and the like, and an irradiation position detector 58 that detects the irradiation position of the spot SP from the subject image. The details of the mode controller 56 will be described later. In a state where the execution of the length measurement mode is allowed, the irradiation position detector 58 detects the irradiation position of the spot SP from the subject image. Specifically, the irradiation position detector 58 calculates the coordinates of the spot SP from the subject image in real time, and obtains the irradiation position of the spot SP from the calculated coordinates. In order to detect the irradiation position of the spot SP by the irradiation position detector 58, a beam color image based on the color of the measurement light needs to be necessarily included in the subject image. It is preferable to acquire the coordinates of the position of the centroid of the spot SP in the subject image as a method of detecting the irradiation position.

The mode controller 56 performs: at least one of a first control that prohibits the switching of a mode to the length measurement mode in a case where an operation for switching a mode to the length measurement mode is performed by the mode changeover switch 13a and a currently set setting condition related to the light source device 14, the endoscope 12, or the processor device 16 corresponds to a predetermined prohibition setting condition; a second control that disables a setting change operation in a case where the setting change operation for the setting condition is performed in the length measurement mode by the user interface 19 and the setting condition to be changed by the setting change operation corresponds to the prohibition setting condition; or a third control that automatically switches a mode to the other mode from the length measurement mode in a case where the setting change operation for the setting condition is performed in the length measurement mode by the user interface 19 and the setting condition to be changed by the setting change operation corresponds to the prohibition setting condition.

The setting condition related to the light source device 14 includes an illumination condition for the illumination light that is used in the normal observation mode or the length measurement mode, an illumination condition for the special light that is used in the special observation mode, or an illumination condition for the measurement light that is used in the length measurement mode. The illumination condition includes, for example, the amount of illumination light and the like. The setting condition related to the endoscope 12 includes an image pickup condition related to the image pickup of the subject. The image pickup condition includes, for example, a shutter speed and the like. The setting condition related to the processor device 16 includes a processing condition, such as image processing related to the subject image. The processing condition includes, for example, color balance, brightness correction, various types of emphasis processing, and the like. In the length measurement mode, it is preferable that the detection of the position of the spotlight SP is optimized and a setting condition (the amount of illumination light, a shutter speed, color balance, brightness correction, and various types of emphasis processing) satisfying visibility at the time of the user's dimensional measurement is set.

The prohibition setting condition includes a first prohibition setting condition that causes the detection of the position of the measurement light-irradiation region from the subject image in the length measurement mode to be hindered, and a second prohibition setting condition that causes the accurate display of a measurement marker corresponding to an observation distance in a measurement image to be hindered. The first prohibition setting condition includes, for example, the special observation mode in which a measurement light image is not used for the display of the measurement image, brightness emphasis or red emphasis in the subject image, and the like. Since a red image as the measurement light image is not used for display on the monitor 18 in the special observation mode as described above, it is difficult to detect the measurement light-irradiation region. It is preferable that the brightness of the subject image is set to be low and redness is suppressed in the length measurement mode as compared to the normal observation mode or the special observation mode.

Further, the second prohibition setting condition includes, for example, the use (ON) of a zoom function, such as the optical zoom function or the digital zoom function. The reason for this is that, since the measurement marker displayed in the measurement image is determined according to the position of the spot SP and is not determined according to the magnification of the zoom function, it is difficult for the measurement marker to be displayed to correspond to an observation distance in a case where the zoom function is ON.

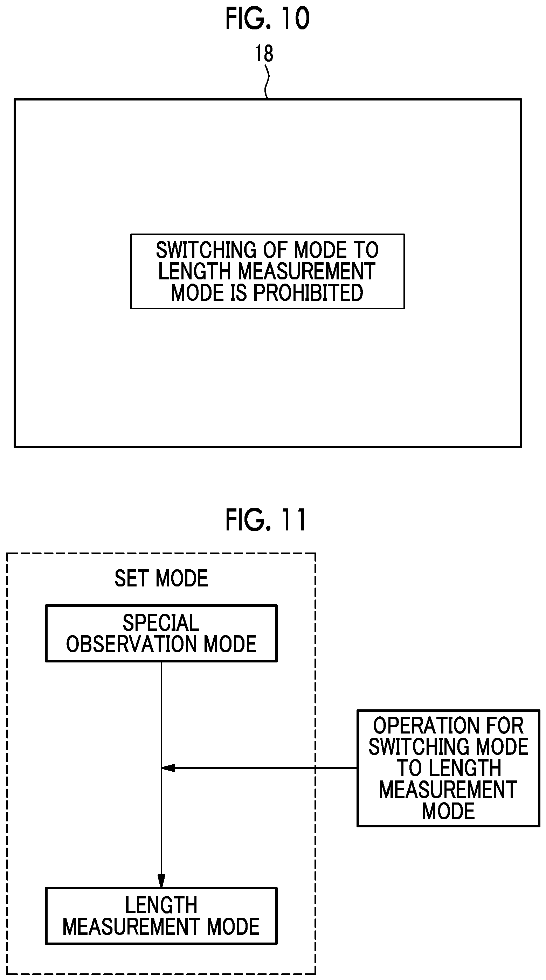

For example, in a case where an operation for switching a mode to the length measurement mode is performed by the mode changeover switch 13a in a state where the endoscope 12 is set to the special observation mode, the mode controller 56 performs the first control that prohibits the switching of a mode to the length measurement mode and maintains the state of the special observation mode as shown in Fig. 9. In a case where the first control is performed, the mode controller 56 displays a message, which notifies that the switching of a mode to the length measurement mode is prohibited, on the monitor 18 as shown in Fig. 10 (warning sound may be made). The mode controller 56 may perform a control to cancel the special observation mode and to switch a mode to the length measurement mode as shown in Fig. 11 instead of prohibiting the switching of a mode to the length measurement mode.

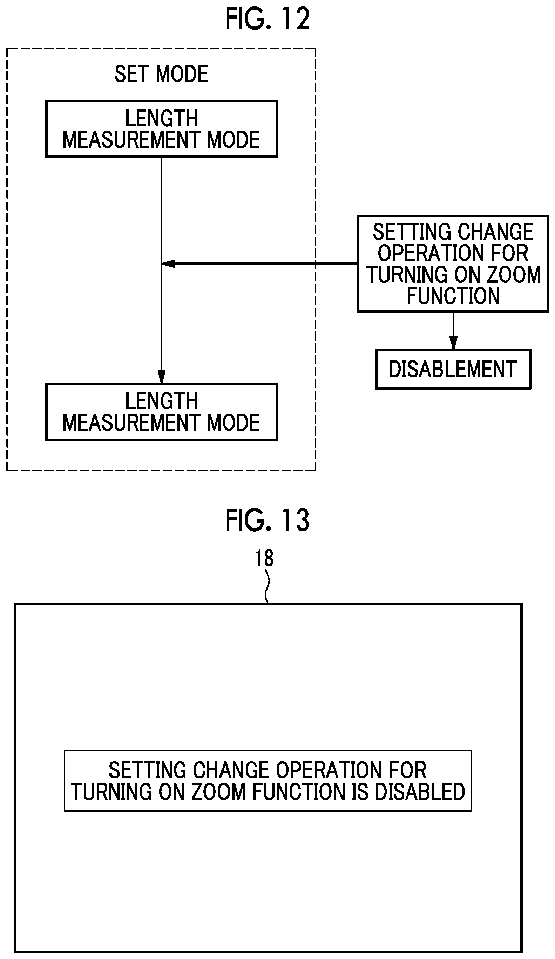

Further, in a case where a setting change operation for turning on the zoom function by the operation of the zoom operation part 13c is performed in the length measurement mode, the mode controller 56 performs the second control that disables the setting change operation for turning on the zoom function as shown in Fig. 12. In a case where the second control is performed, the mode controller 56 displays a message, which notifies that the setting change operation for turning on the zoom function is disabled, on the monitor 18 as shown in Fig. 13 (warning sound may be made).

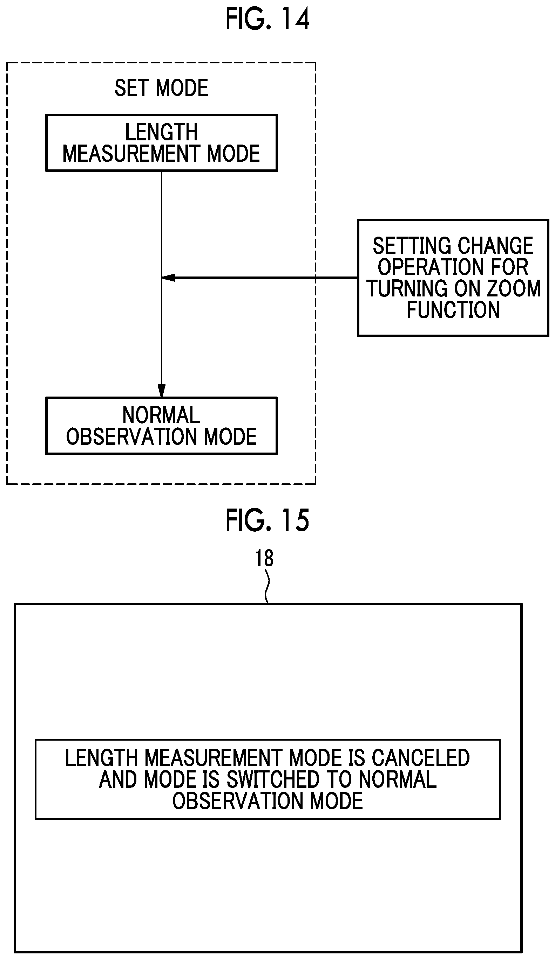

Furthermore, in a case where a setting change operation for turning on the zoom function by the operation of the zoom operation part 13c is performed in the length measurement mode, the mode controller 56 performs the third control that cancels the length measurement mode and switches a mode to the normal observation mode as the other mode as shown in Fig. 14. In a case where the third control is performed, the mode controller 56 displays a message, which notifies that the length measurement mode is canceled (the measurement marker is not displayed) and is switched to the normal observation mode, on the monitor 18 as shown in Fig. 15 (warning sound may be made). In a case where the third control is performed, the setting change operation for turning on the zoom function is enabled and the subject in the subject image is enlarged or reduced by the zoom function.

The second signal processing unit 52 sets a first measurement marker as a measurement marker, which is used to measure the size of the subject, on the basis of the irradiation position of the spot SP and sets the first measurement marker at a marker display position displayed on the monitor 18. The second signal processing unit 52 sets a measurement marker image corresponding to the irradiation position with reference to a marker table 62 in which the measurement marker image of which the display aspect varies depending on the irradiation position of the spot SP and the marker display position is stored in association with the irradiation position of the spot.

For example, the size or shape of the measurement marker image varies depending on the irradiation position of the spot SP and the marker display position. The display of the measurement marker image will be described later. Further, the contents stored in the marker table 62 are maintained even in a case where the power of the processor device 16 is turned off. The measurement marker image and the irradiation position are stored in the marker table 62 in association with each other, but a distance to the subject (a distance between the distal end part 12d of the endoscope 12 and the subject) corresponding to the irradiation position and the measurement marker image may be stored in the marker table 62 in association with each other.

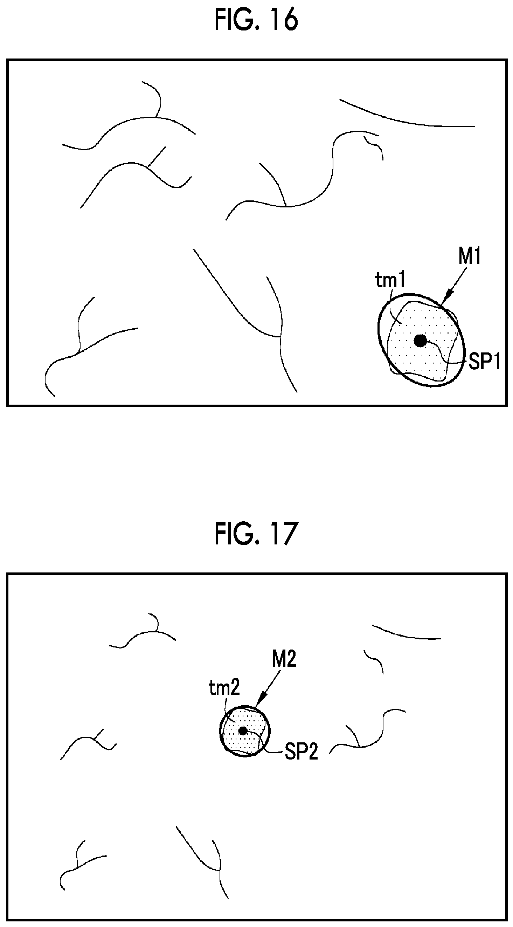

In a case where the measurement image in which the measurement marker is superimposed on the subject image is displayed on the monitor 18, the display controller 40 performs a control to cause the display aspect of the measurement marker to vary depending on the irradiation position of the spot SP and the marker display position. Specifically, the display controller 40 causes the monitor 18 to display the measurement image in which the first measurement marker is superimposed to center on the spot SP. For example, a circular measurement marker is used as the first measurement marker. In this case, as shown in Fig. 16, a marker M1, which shows an actual size of 5 mm (a horizontal direction and a vertical direction of the subject image), is displayed at the center of a spot SP1 formed on a tumor tm1 of a subject in a case where an observation distance is close to the near end Px. In a case where the measurement marker is displayed on the monitor 18, an observation distance may also be displayed on the monitor 18 together.

Since the marker display position of the marker M1 is positioned at the peripheral portion of the subject image that is affected by distortion caused by the image pickup optical system 21, the marker M1 has an elliptical shape due to an influence of the distortion or the like. Since the above-mentioned marker M1 substantially coincides with the range of the tumor tm1, the size of the tumor tm1 can be measured as about 5 mm. In the subject image, the spot may not be displayed and only the first measurement marker may be displayed.

Further, as shown in Fig. 17, a marker M2, which shows an actual size of 5 mm (the horizontal direction and the vertical direction of the subject image), is displayed at the center of a spot SP2 formed on a tumor tm2 of a subject in a case where an observation distance is close to the intermediate vicinity Py. Since the marker display position of the marker M2 is positioned at the central portion of the subject image that is less likely to be affected by distortion caused by the image pickup optical system 21, the marker M2 has a circular shape without being affected by the distortion or the like.

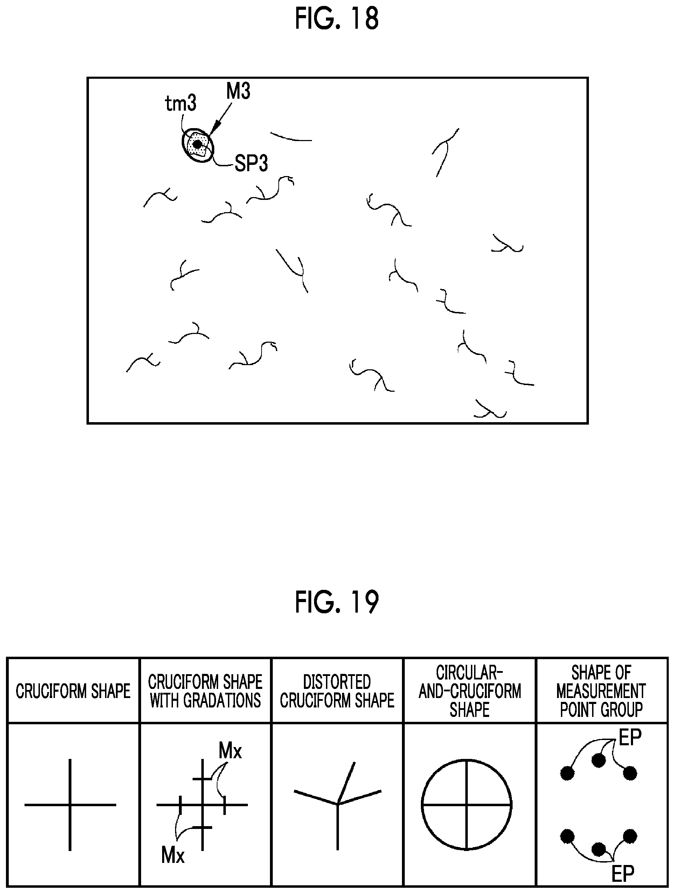

Furthermore, as shown in Fig. 18, a marker M3, which shows an actual size of 5 mm (the horizontal direction and the vertical direction of the subject image), is displayed at the center of a spot SP3 formed on a tumor tm3 of a subject. Since the marker display position of the marker M3 is positioned at the peripheral portion of the subject image that is affected by distortion caused by the image pickup optical system 21, the marker M3 has an elliptical shape due to an influence of the distortion or the like. As shown in Figs. 16 to 18 having been described above, the size of the first measurement marker corresponding to the same actual size of 5 mm is reduced with an increase in an observation distance. Moreover, the shape of the first measurement marker also varies depending on the marker display position due to an influence of the distortion caused by the image pickup optical system 21.

In Figs. 16 to 18, the center of the spot SP and the center of the marker are displayed to coincide with each other. However, the first measurement marker may be displayed at a position away from the spot SP in a case where there is no problem in measurement accuracy. Even in this case, it is preferable that the first measurement marker is displayed near the spot. Further, the distorted first measurement marker is not displayed, and the distortion of the subject image may be corrected so that an undistorted first measurement marker may be displayed in a corrected subject image.

Furthermore, the first measurement marker corresponding to the actual size of the subject, which is 5 mm, is displayed in Figs. 16 to 18, but the actual size of the subject may be set to any value (for example, 2 mm, 3 mm, 10 mm, or the like) according to an object to be observed or the purpose of observation. Further, the first measurement marker has a substantially circular shape in Figs. 16 to 18, but may have a cruciform shape where a vertical line and a horizontal line intersect with each other as shown in Fig. 19. Furthermore, the first measurement marker may have a cruciform shape with gradations where gradations Mx are given to at least one of a vertical line or a horizontal line of a cruciform shape. Further, the first measurement marker may have a distorted cruciform shape of which at least one of a vertical line or a horizontal line is inclined. Furthermore, the first measurement marker may have a circular-and-cruciform shape where a cruciform shape and a circle are combined with each other. In addition, the first measurement marker may have the shape of a measurement point group where a plurality of measurement points EP corresponding to an actual size from a spot are combined with each other. Further, one first measurement marker may be displayed or a plurality of first measurement markers may be displayed, and the color of the first measurement marker may be changed according to an actual size.

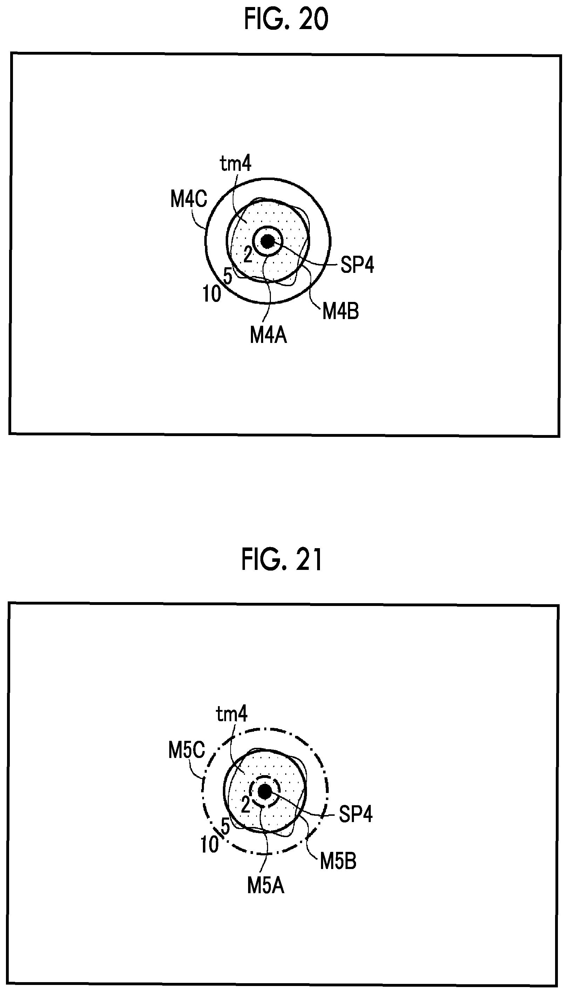

As shown in Fig. 20, (three) circular markers M4A, M4B, and M4C having different sizes (diameters as the sizes are 2 mm, 5 mm, and 10 mm, respectively) may be displayed in the subject image as the first measurement marker to center on a spot SP4 formed on a tumor tm4. Since the three concentric circular markers are displayed as a plurality of markers, time and effort required to switch a marker can be saved and measurement can be performed even in a case where a subject has a non-linear shape. In a case where a plurality of concentric circular markers are to be displayed to center on a spot, a size and a color are not designated for each marker and combinations of a plurality of conditions may be prepared in advance and one can be selected from these combinations. Further, the first measurement marker is not limited to three circular markers as shown in Fig. 20, and two or four or more circular markers may be used. The three circular markers are concentric circles having the same center in Fig. 20, but may be a plurality of circles based on the position of the spot SP4 without being limited to concentric circles.

In Fig. 20, all the three concentric circular markers are displayed with the same color (black). However, in a case where a plurality of concentric circular markers are to be displayed, a plurality of color concentric circular markers of which colors are different from each other may be used. As shown in Fig. 21, a marker M5A is displayed by a dotted line representing a red color, a marker M5B is displayed by a solid line representing a blue color, and a marker M5C is displayed by a one-dot chain line representing a white color. Since identifiability can be improved in a case where the colors of the markers are different from each other in this way, measurement can be easily performed.

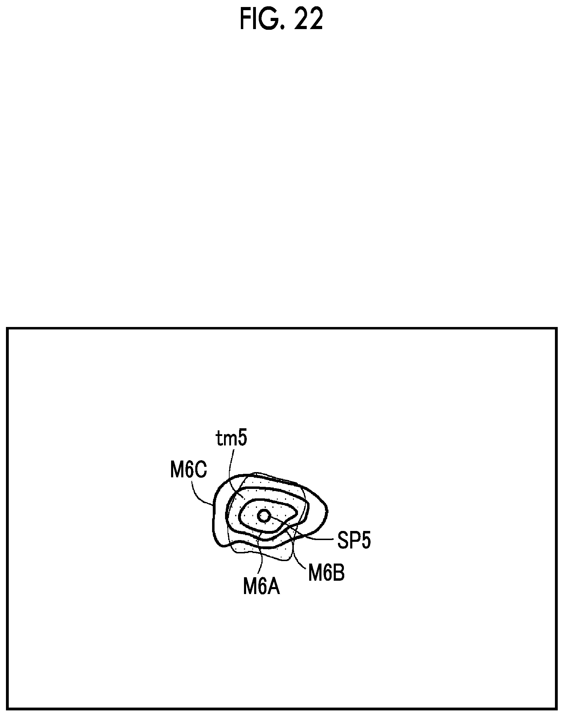

Further, as shown in Fig. 22, a plurality of distorted concentric circular markers, which are distorted from the respective concentric circles, may be used as the first measurement marker other than the plurality of concentric circular markers. In this case, distorted concentric circular markers M6A, M6B, and M6C are displayed in the subject image to center on a spot SP5 formed on a tumor tm5.

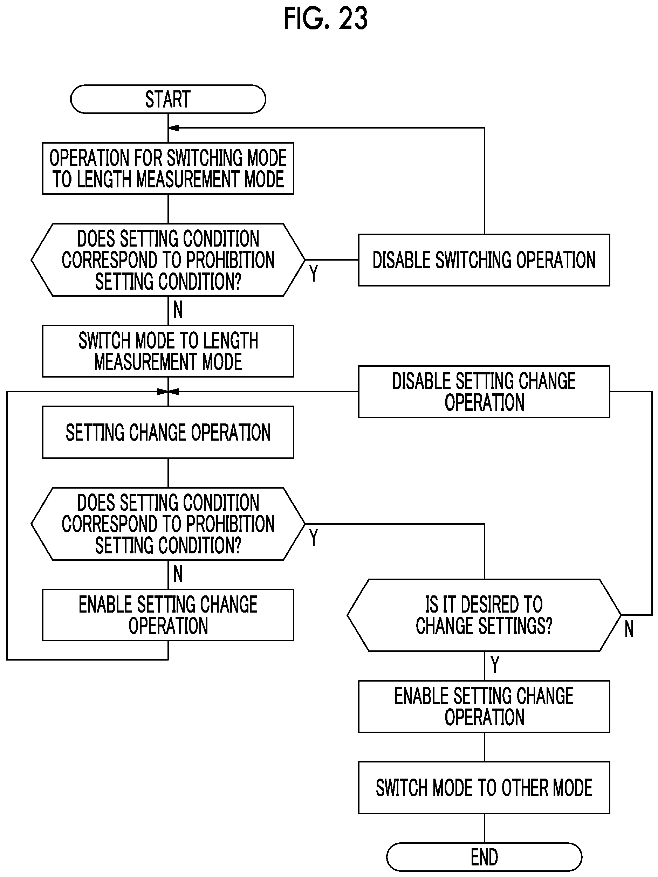

Next, a series of flows of the invention will be described with reference to a flowchart shown in Fig. 23. In a case where a user operates the mode changeover switch 13a to perform an operation for switching a mode to the length measurement mode and a currently set setting condition related to the light source device 14, the endoscope 12, or the processor device 16 corresponds to a preset prohibition setting condition, the mode controller 56 performs the first control that disables the operation for switching a mode to the length measurement mode and prohibits the switching of a mode to the length measurement mode. In a case where the first control is performed, a message notifying "the switching of a mode to the length measurement mode is prohibited" is displayed on the monitor 18. In a case where the user desires to use the length measurement mode, the user operates the mode changeover switch 13a, the user interface 19, or the like to perform an operation for changing a condition to a setting condition not corresponding to the prohibition setting condition. Then, the user operates the mode changeover switch 13a again to perform an operation for switching a mode to the length measurement mode. Accordingly, the switching of a mode to the length measurement mode is performed.

After that, in a case where the setting change operation for the setting condition is performed in the length measurement mode by the user interface 19 or the like and the setting condition to be changed by the setting change operation corresponds to the prohibition setting condition, the mode controller 56 performs the second control that disables the setting change operation and maintains the length measurement mode. In a case where the second control is performed, a message notifying "the setting change operation is disabled" is displayed on the monitor 18.

On the other hand, in a case where a user desires to cancel the length measurement mode and to enable the setting change operation even though a setting condition to be changed by the setting change operation corresponds to the prohibition setting condition in the length measurement mode, the user operates the user interface 19 or the like to perform the third control that switches a mode to the other mode from the length measurement mode. In a case where the third control is performed, the setting condition for which the setting change operation is enabled is changed. Further, a message notifying "a mode is switched to the other mode from the length measurement mode" is displayed on the monitor 18. Icons, indicators, or the like, which show the states of the length measurement mode or observation conditions, may be used as these notifications.

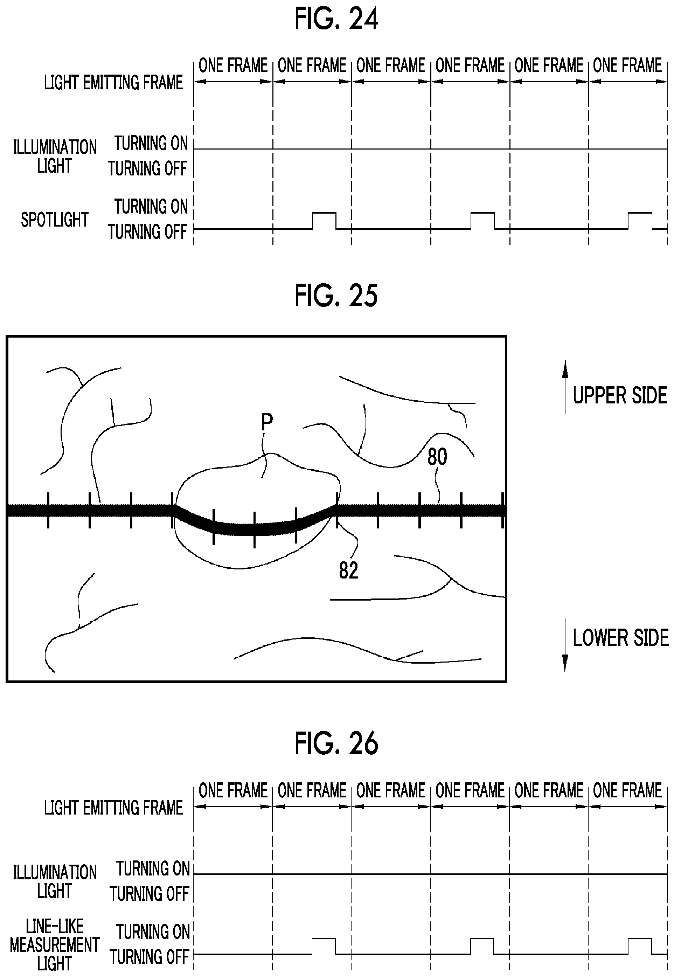

The subject is constantly irradiated with the illumination light and spotlight (measurement light) in the length measurement mode. However, as shown in Fig. 24, the illumination light may be constantly turned on so that the subject is irradiated with the illumination light and the spotlight may be repeatedly turned on and turned off (dimmed) every frame (or every few frames) so that the subject is intermittently irradiated with the spotlight. In this case, in a frame where the spotlight is turned on, the position of the spotlight is detected and the display of a measurement marker is set. Further, it is preferable that the measurement marker of which the display is set is superimposed and displayed on an image obtained in a frame where the subject is irradiated with only the illumination light.

Light, which forms a spot in a case where a subject is irradiated with the light, is used as the measurement light, but other light may be used. For example, line-like measurement light, which is formed on the subject as an intersection line 80 as shown in Fig. 25 in a case where the subject is irradiated with the light, may be used. In a case where the subject is irradiated with the line-like measurement light, the intersection line 80 as a line-like irradiation region is formed on the subject. In this case, a second measurement marker that consists of the intersection line 80 and gradations 82 formed on the intersection line 80 and serving as an index of the size of the subject (for example, a polyp P) is generated as a measurement marker. In a case where the line-like measurement light is used, the irradiation position detector 58 detects the position of the intersection line 80 (the irradiation position of the measurement light). An observation distance is shorter as the intersection line 80 is positioned closer to the lower side, and an observation distance is longer as the intersection line 80 is positioned closer to the upper side. For this reason, an interval between the gradations 82 is larger as the intersection line 80 is positioned closer to the lower side, and an interval between the gradations 82 is smaller as the intersection line 80 is positioned closer to the upper side.

In a case where the line-like measurement light is used as the measurement light, in the length measurement mode, the subject may be constantly irradiated with the illumination light and the line-like measurement light or, as shown in Fig. 26, the subject may be constantly irradiated with the illumination light and the line-like measurement light may be repeatedly turned on and turned off (dimmed) every frame (or every few frames) so that the subject is intermittently irradiated with the line-like measurement light. In this case, in a frame where the line-like measurement light is turned on, the position of the line-like measurement light is detected and the display of a measurement marker is set. Further, it is preferable that the measurement marker of which the display is set is superimposed and displayed on an image obtained in a frame where the subject is irradiated with only the illumination light.

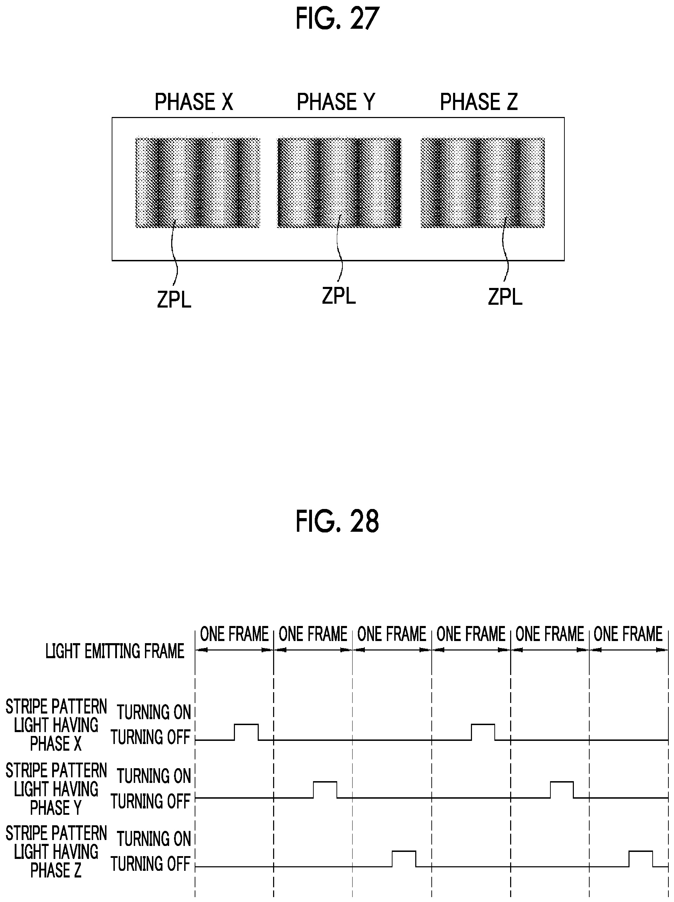

Stripe pattern light ZPL, which is formed as light having a stripe pattern on a subject as shown in Fig. 27 in a case where the subject is irradiated with the light, may be used as the measurement light (for example, see JP2016-198304A). The stripe pattern light ZPL is obtained in a case where a variable-transmittance liquid crystal shutter (not shown) is irradiated with specific laser light, and is formed of two different vertical stripe patterns in which a region (transmissive region) through which specific laser light is transmitted by the liquid crystal shutter and a region (non-transmissive region) through which the specific laser light is not transmitted are periodically repeated in the horizontal direction. In a case where the stripe pattern light is used as the measurement light, the period of the stripe pattern light is changed depending on a distance from the subject. Accordingly, the subject is irradiated with the stripe pattern light plural times while the period or phase of the stripe pattern light is shifted by the liquid crystal shutter, and the three-dimensional shape of the subject is measured on the basis of a plurality of images obtained from the shift of the period or phase.

For example, a subject is alternately irradiated with a stripe pattern light having a phase X, a stripe pattern light having a phase Y, and a stripe pattern light having a phase Z. The phases of the vertical stripe patterns of the stripe pattern lights having the phases X, Y, and Z are shifted from each other by 120° (2π/3). In this case, the three-dimensional shape of the subject is measured using three types of images obtained on the basis of the respective stripe pattern lights. For example, it is preferable that the subject is irradiated with the stripe pattern light having the phase X, the stripe pattern light having the phase Y, and the stripe pattern light having the phase Z while the stripe pattern light having the phase X, the stripe pattern light having the phase Y, and the stripe pattern light having the phase Z are switched every frame (or every few frames) as shown in Fig. 28. It is preferable that the subject is constantly irradiated with the illumination light.

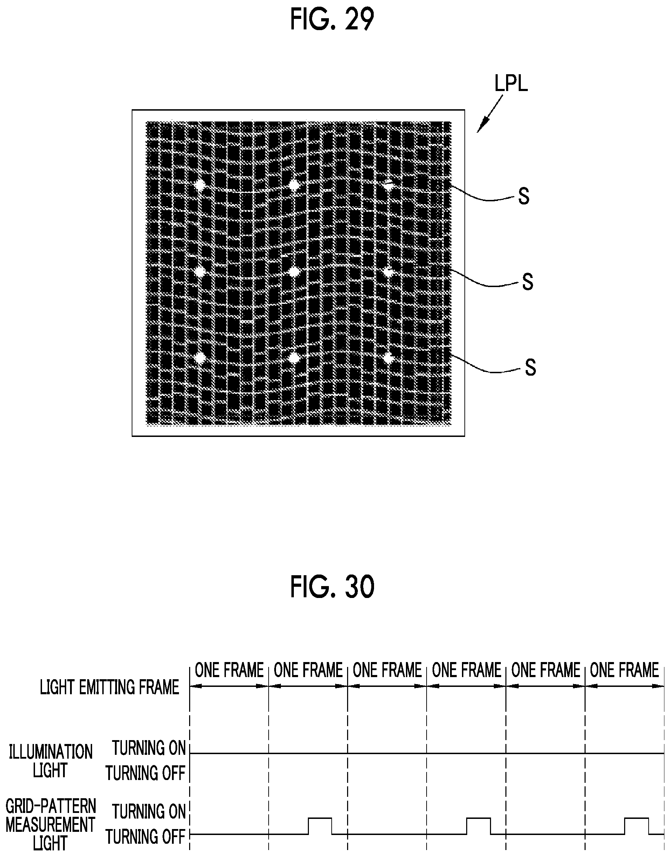

Grid-pattern measurement light LPL, which is formed as a grid pattern as shown in Fig. 29 in a case where a subject is irradiated with the light, may be used as the measurement light (for example, see JP2017-217215A). In this case, since the three-dimensional shape of the subject is measured according to the state of deformation of the grid pattern in a case where the subject is irradiated with the grid-pattern measurement light LPL, it is required to accurately detect the grid pattern. For this reason, the shape of the grid-pattern measurement light LPL is not a perfect grid shape and is set to a wave shape or the like, that is, is slightly deformed from a grid shape to improve the detection accuracy of the grid pattern. Further, the grid pattern is provided with S codes indicating that the end points of the left and right horizontal lines are continuous. In a case where the grid pattern is detected, not only the pattern but also the S codes are detected to improve the detection accuracy of the pattern. The grid pattern may be a pattern in which a plurality of spots are arranged in a grid shape in the vertical and horizontal directions in addition to a pattern in which vertical lines and horizontal lines are regularly arranged.

In a case where the grid-pattern measurement light LPL is used as the measurement light, in the length measurement mode, the subject may be constantly irradiated with the illumination light and the grid-pattern measurement light LPL or, as shown in Fig. 30, the subject may be constantly irradiated with the illumination light and the grid-pattern measurement light LPL may be repeatedly turned on and turned off (dimmed) every frame (or every few frames) so that the subject is intermittently irradiated with the grid-pattern measurement light LPL. In this case, the three-dimensional shape is measured on the basis of the grid-pattern measurement light LPL in a frame where the grid-pattern measurement light LPL is turned on. Further, it is preferable that the measurement result of the three-dimensional shape is superimposed and displayed on an image obtained in a frame where the subject is irradiated with only the illumination light.

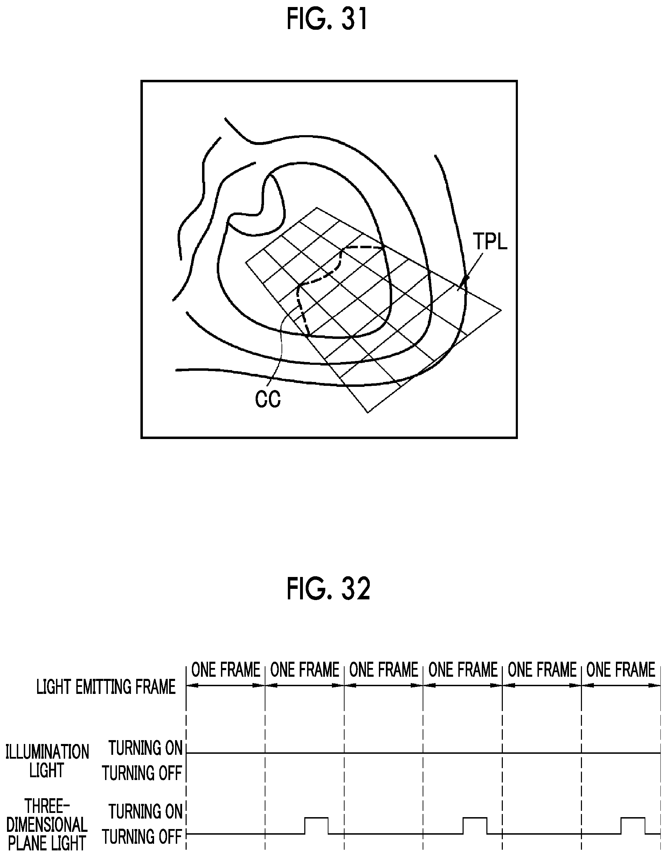

Three-dimensional plane light TPL, which is represented in a subject image by mesh lines as shown in Fig. 31, may be used as the measurement light (for example, see JP2017-508529A). In this case, a user moves the distal end part 12d so that the three-dimensional plane light TPL is aimed at an object to be measured. Then, in a case where the three-dimensional plane light TPL intersects with the object to be measured, a distance from an intersection curve CC between the three-dimensional plane light TPL and the subject is calculated by processing based on a manual operation of the user interface or the like, or automatic processing.

In a case where the three-dimensional plane light TPL is used as the measurement light, in the length measurement mode, the subject may be constantly irradiated with the illumination light and the three-dimensional plane light TPL or, as shown in Fig. 32, the subject may be constantly irradiated with the illumination light and the three-dimensional plane light TPL may be repeatedly turned on and turned off (dimmed) every frame (or every few frames) so that the subject is intermittently irradiated with the three-dimensional plane light TPL.

In the embodiment, the hardware structures of processing units, which perform various types of processing, such as the reception unit 38, the signal processing unit 39, the display controller 40, the system controller 41, the static image storage unit 42, the first signal processing unit 50, the second signal processing unit 52, the mode controller 56, the irradiation position detector 58, and the marker table 62, are various processors to be described below. Various processors include: a central processing unit (CPU) that is a general-purpose processor functioning as various processing units by executing software (program); a programmable logic device (PLD) that is a processor of which the circuit configuration can be changed after manufacture, such as a field programmable gate array (FPGA); a dedicated electrical circuit that is a processor having circuit configuration designed exclusively to perform various types of processing; and the like.

One processing unit may be formed of one of these various processors, or may be formed of a combination of two or more same kind or different kinds of processors (for example, a plurality of FPGAs, or a combination of a CPU and an FPGA). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined. Further, the hardware structure of the storage unit is a storage device, such as a hard disc drive (HDD) or a solid state drive (SSD).

It is preferable that the measurement light and irradiation with the measurement light are as follows. It is preferable that the measurement light is spotlight. It is preferable that the measurement light is line-like measurement light. It is preferable that the measurement light is grid-pattern measurement light. It is preferable that the measurement light is three-dimensional plane light. It is preferable that the subject is intermittently irradiated with the measurement light. It is preferable that the measurement light is stripe pattern light. It is preferable that the subject is irradiated with a plurality of types of stripe pattern lights having different phases or periods while the plurality of types of stripe pattern lights are switched.

### Explanation of References

10: endoscope system
12: endoscope
12a: insertion part
12b: operation part
12c: bendable part
12d: distal end part
13a: mode changeover switch
13b: freeze switch
13c: zoom operation part
14: light source device
16: processor device
18: monitor
19: user interface
21: image pickup optical system
21a: objective lens
21b: zoom lens
22: illumination optical system
22a: illumination lens
23: measurement light-emitting unit
23a: light source
23b: DOE
23c: prism
23d: emitting section
24: opening
25: air/water supply nozzle
26: light source unit
27: light source controller
28: light guide
29: connector
32: image pickup element
33: image pickup controller
34: CDS/AGC circuit
35: A/D converter
36: communication I/F
37: communication I/F
38: reception unit
39: signal processing unit
40: display controller
41: system controller
42: static image storage unit
50: first signal processing unit
52: second signal processing unit
56: mode controller
58: irradiation position detector
62: marker table
80: intersection line
82: gradation
101, 102: solid line
M1, M2, M3: cruciform marker
tm1, tm2, tm3, tm4, tm5: tumor
SP: spot
SP1, SP2, SP3, SP4, SP5: spot
M4A, M4B, M4C, M5A, M5B, M5C: circular marker
M6A, M6B, M6C: distorted concentric circular marker
P: polyp
M1, M2, M3: cruciform marker
tm1, tm2, tm3, tm4, tm5: tumor
SP: spot
SP1, SP2, SP3, SP4, SP5: spot
M4A, M4B, M4C, M5A, M5B, M5C: circular marker
M6A, M6B, M6C: distorted concentric circular marker
P: polyp

## Claims

1. An endoscope system comprising:
a light source device (14);
an endoscope (12) configured to irradiate a subject with measurement light; and
a processor device (16) that includes a processor and is configured to generate a subject image related to the subject,
wherein the processor is configured to perform at least one of
a first control that prohibits switching of a mode to a length measurement mode in which a measurement image in which a measurement marker used to measure a size of the subject is displayed is displayed on a display in a case where an operation for switching a mode to the length measurement mode is performed and a setting condition related to the light source device, the endoscope, or the processor device corresponds to a predetermined prohibition setting condition;
a second control that disables a setting change operation for the setting condition in a case where the setting change operation is performed in the length measurement mode and the setting condition to be changed to by the setting change operation corresponds to the prohibition setting condition; or
a third control that switches a mode to another mode from the length measurement mode in a case where the setting change operation for the setting condition is performed in the length measurement mode and the setting condition to be changed to by the setting change operation corresponds to the prohibition setting condition;
wherein the endoscope (12) includes a measurement light-emitting unit (23) that emits the measurement light and an image pickup optical system that is provided in a distal end part at a position different from a position of the measurement light-emitting unit and receives light from the subject, and the measurement light is emitted obliquely with respect to an optical axis of the image pickup optical system, and
the processor is configured to acquire the subject image that includes a measurement light-irradiation region appearing on the subject due to irradiation with the measurement light, detect a position of the measurement light-irradiation region from the subject image, and display the measurement image on a display, wherein the measurement marker is set according to the position of the measurement light-irradiation region.

2. The endoscope system according to claim 1,
wherein the prohibition setting condition includes a first prohibition setting condition that causes detection of a position of a measurement light-irradiation region, which appears on the subject due to irradiation with the measurement light, from the subject image to be hindered.

3. The endoscope system according to claim 2,
wherein the subject image includes a measurement light image based on a color of the measurement light,
the setting condition includes a special observation mode in which the measurement light image is not used for display of the measurement image, and
the first prohibition setting condition includes the special observation mode.

4. The endoscope system according to claim 3,
wherein the processor is configured to perform a control to cancel the special observation mode and to switch a mode to the length measurement mode instead of prohibiting switching of a mode to the length measurement mode in a case where an operation for switching a mode to the length measurement mode is performed in the special observation mode.

5. The endoscope system according to claim 3 or 4,
wherein the measurement light image is a red image.

6. The endoscope system according to any one of claims 1 to 5,
wherein the prohibition setting condition includes a second prohibition setting condition that causes display of a measurement marker corresponding to an observation distance in the measurement image to be hindered.

7. The endoscope system according to claim 6,
wherein the second prohibition setting condition includes a use of a zoom function that enlarges or reduces the subject in the subject image.

8. The endoscope system according to any one of claims 1 to 7,
wherein the processor is configured to notify that the switching of a mode to the length measurement mode is prohibited in a case where the first control for prohibiting the switching of a mode to the length measurement mode is performed.

9. The endoscope system according to any one of claims 1 to 7,
wherein the processor is configured to notify that the setting change operation is disabled in a case where the second control for disabling the setting change operation is performed.

10. The endoscope system according to any one of claims 1 to 7,
wherein the processor is configured to notify that a mode is switched to another mode in a case where the third control for switching a mode to another mode from the length measurement mode is performed.

11. The endoscope system according to any one of claims 1 to 10,
wherein the measurement marker includes a first measurement marker showing an actual size of the subject or a second measurement marker consisting of an intersection line formed on the subject by the measurement light and gradations formed on the intersection line and serving as an index of the size of the subject.

12. A method of operating a processor of an endoscope system, the endoscope system including a light source device (14), an endoscope (12) that is configured to irradiate a subject with measurement light, and a processor device (16) that includes the processor and is configured to generate a subject image related to the subject,
wherein the processor performs at least one of
a first control that prohibits switching of a mode to a length measurement mode in which a measurement image in which a measurement marker used to measure a size of the subject is displayed is displayed on a display in a case where an operation for switching a mode to the length measurement mode is performed and a setting condition related to the light source device, the endoscope, or the processor device corresponds to a predetermined prohibition setting condition;
a second control that disables a setting change operation for the setting condition in a case where the setting change operation is performed in the length measurement mode and the setting condition to be changed to by the setting change operation corresponds to the prohibition setting condition; or
a third control that switches a mode to another mode from the length measurement mode in a case where the setting change operation for the setting condition is performed in the length measurement mode and the setting condition to be changed to by the setting change operation corresponds to the prohibition setting condition;
wherein the endoscope (12) includes a measurement light-emitting unit (23) that emits the measurement light and an image pickup optical system that is provided in a distal end part at a position different from a position of the measurement light-emitting unit and receives light from the subject, and the measurement light is emitted obliquely with respect to an optical axis of the image pickup optical system, and
the processor acquires the subject image that includes a measurement light-irradiation region appearing on the subject due to irradiation with the measurement light, detects a position of the measurement light-irradiation region from the subject image, and displays the measurement image on a display, wherein the measurement marker is set according to the position of the measurement light-irradiation region.

## Patentansprüche

1. Endoskopsystem, umfassend:
eine Lichtquellenvorrichtung (14);
ein Endoskop (12), das so konfiguriert ist, dass es ein Motiv mit Messlicht bestrahlt; und
eine Prozessorvorrichtung (16), die einen Prozessor enthält und so konfiguriert ist, dass sie ein Motivbild, das sich auf das Motiv bezieht, erzeugt,
wobei der Prozessor so konfiguriert ist, dass er durchführt, mindestens eine von einer ersten Steuerung, die Umschalten eines Modus auf einen Längenmessmodus, bei dem ein Messbild, in dem eine Messmarkierung, die zum Messen einer Größe des Motivs verwendet wird, angezeigt wird, auf einer Anzeige angezeigt wird, in einem Fall, in dem eine Betätigung zum Umschalten eines Modus auf den Längenmessmodus durchgeführt wird und eine Einstellungsbedingung, die sich auf die Lichtquellenvorrichtung, das Endoskop oder die Prozessorvorrichtung bezieht, einer vorbestimmten Verbotseinstellungsbedingung entspricht, verbietet;
einer zweiten Steuerung, die eine Einstellungsänderungsbetätigung für die Einstellungsbedingung in einem Fall, in dem die Einstellungsänderungsbetätigung in dem Längenmessmodus durchgeführt wird und die durch die Einstellungsänderungsbetätigung zu ändernde Einstellungsbedingung der Verbotseinstellungsbedingung entspricht, deaktiviert; oder
einer dritten Steuerung, die einen Modus in einem Fall, in dem die Einstellungsänderungsbetätigung für die Einstellungsbedingung in dem Längenmessmodus durchgeführt wird und die Einstellungsbedingung, die durch die Einstellungsänderungsbetätigung zu ändern ist, der Verbotseinstellungsbedingung entspricht, von dem Längenmessmodus auf einen anderen Modus umschaltet;
wobei das Endoskop (12) eine Messlicht emittierende Einheit (23), die das Messlicht emittiert, und ein optisches System für Bildaufnahme, das in einem distalen Endteil an einer Position, die sich von einer Position der Messlicht emittierenden Einheit unterscheidet, vorgesehen ist und Licht von dem Motiv empfängt, enthält und das Messlicht in Bezug auf eine optische Achse des optischen Systems für Bildaufnahme schräg emittiert wird, und
der Prozessor so konfiguriert ist, dass er das Motivbild, das einen Messlicht-Bestrahlungsbereich, der aufgrund von Bestrahlung mit dem Messlicht auf dem Motiv erscheint, enthält, erfasst, eine Position des Messlicht-Bestrahlungsbereichs aus dem Motivbild detektiert und das Messbild auf einem Anzeige anzeigt, wobei die Messmarkierung gemäß der Position des Messlicht-Bestrahlungsbereichs eingestellt ist.

2. Endoskopsystem nach Anspruch 1,
wobei die Verbotseinstellungsbedingung eine erste Verbotseinstellbedingung umfasst, die veranlasst, dass Detektion einer Position eines Messlicht-Bestrahlungsbereichs, der aufgrund von Bestrahlung mit dem Messlicht auf dem Motiv erscheint, aus dem Motivbild behindert wird.

3. Endoskopsystem nach Anspruch 2,
wobei das Motivbild ein Messlichtbild, das auf einer Farbe des Messlichts basiert, umfasst,
die Einstellungsbedingung einen speziellen Beobachtungsmodus, bei dem das Messlichtbild nicht zur Anzeige des Messbildes verwendet wird, umfasst, und
die erste Verbotseinstellungsbedingung den speziellen Beobachtungsmodus umfasst.

4. Endoskopsystem nach Anspruch 3,
wobei der Prozessor so konfiguriert ist, dass er eine Steuerung zum Abbrechen des speziellen Beobachtungsmodus und zum Umschalten eines Modus auf den Längenmessmodus anstelle von Verbieten von Umschalten eines Modus auf den Längenmessmodus in einem Fall, in dem eine Betätigung zum Umschalten eines Modus auf den Längenmessmodus in dem speziellen Beobachtungsmodus durchgeführt wird, durchführt.

5. Endoskopsystem nach Anspruch 3 oder 4,
wobei das Messlichtbild ein rotes Bild ist.

6. Endoskopsystem nach einem der Ansprüche 1 bis 5,
wobei die Verbotseinstellungsbedingung eine zweite Verbotseinstellungsbedingung umfasst, die veranlasst, dass Anzeige einer Messmarkierung, die einer Beobachtungsentfernung in dem Messbild entspricht, behindert wird.

7. Endoskopsystem nach Anspruch 6,
wobei die zweite Verbotseinstellungsbedingung eine Verwendung einer Zoomfunktion, die das Motiv in dem Motivbild vergrößert oder verkleinert, umfasst.

8. Endoskopsystem nach einem der Ansprüche 1 bis 7,
wobei der Prozessor so konfiguriert ist, dass er darüber benachrichtigt, dass das Umschalten eines Modus auf den Längenmessmodus in einem Fall, in dem die erste Steuerung zum Verbieten des Umschaltens eines Modus auf den Längenmessmodus durchgeführt wird, verboten ist.

9. Endoskopsystem nach einem der Ansprüche 1 bis 7,
wobei der Prozessor so konfiguriert ist, dass er darüber benachrichtigt, dass die Einstellungsänderungsbetätigung in einem Fall, in dem die zweite Steuerung zum Deaktivieren der Einstellungsänderungsbetätigung durchgeführt wird, deaktiviert ist.

10. Endoskopsystem nach einem der Ansprüche 1 bis 7,
wobei der Prozessor so konfiguriert ist, dass er darüber benachrichtigt, dass ein Modus auf einen anderen Modus in einem Fall, in dem die dritte Steuerung zum Umschalten eines Modus von dem Längenmessmodus auf einen anderen Modus durchgeführt wird, umgeschaltet wird.

11. Endoskopsystem nach einem der Ansprüche 1 bis 10,
wobei die Messmarkierung eine erste Messmarkierung, die eine tatsächliche Größe des Motivs zeigt, oder eine zweite Messmarkierung, die aus einer Schnittlinie, die durch das Messlicht auf dem Motiv gebildet ist, und Abstufungen, die an der Schnittlinie gebildet sind und als ein Index der Größe des Motivs dienen, besteht, umfasst.

12. Verfahren des Betreibens eines Prozessors eines Endoskopsystems, wobei das Endoskopsystem eine Lichtquellenvorrichtung (14), ein Endoskop (12), das so konfiguriert ist, dass es ein Motiv mit Messlicht bestrahlt, und eine Prozessorvorrichtung (16), die den Prozessor enthält und so konfiguriert ist, dass sie ein Motivbild, das sich auf das Motiv bezieht, erzeugt, enthält,
wobei der Prozessor durchführt, mindestens eine von
einer ersten Steuerung, die Umschalten eines Modus auf einen Längenmessmodus, bei dem ein Messbild, in dem eine Messmarkierung, die zum Messen einer Größe des Motivs verwendet wird, angezeigt wird, auf einer Anzeige angezeigt wird, in einem Fall, in dem eine Betätigung zum Umschalten eines Modus auf den Längenmessmodus durchgeführt wird und eine Einstellungsbedingung, die sich auf die Lichtquellenvorrichtung, das Endoskop oder die Prozessorvorrichtung bezieht, einer vorbestimmten Verbotseinstellungsbedingung entspricht, verbietet;
einer zweiten Steuerung, die eine Einstellungsänderungsbetätigung für die Einstellungsbedingung in einem Fall, in dem die Einstellungsänderungsbetätigung in dem Längenmessmodus durchgeführt wird und die durch die Einstellungsänderungsbetätigung zu ändernde Einstellungsbedingung der Verbotseinstellungsbedingung entspricht, deaktiviert; oder
einer dritten Steuerung, die einen Modus in einem Fall, in dem die Einstellungsänderungsbetätigung für die Einstellungsbedingung in dem Längenmessmodus durchgeführt wird und die Einstellungsbedingung, die durch die Einstellungsänderungsbetätigung zu ändern ist, der Verbotseinstellungsbedingung entspricht, von dem Längenmessmodus auf einen anderen Modus umschaltet;
wobei das Endoskop (12) eine Messlicht emittierende Einheit (23), die das Messlicht emittiert, und ein optisches System für Bildaufnahme, das in einem distalen Endteil an einer Position, die sich von einer Position der Messlicht emittierenden Einheit unterscheidet, vorgesehen ist und Licht von dem Motiv empfängt, enthält und das Messlicht in Bezug auf eine optische Achse des optischen Systems für Bildaufnahme schräg emittiert wird, und
der Prozessor das Motivbild, das einen Messlicht-Bestrahlungsbereich, der aufgrund von Bestrahlung mit dem Messlicht auf dem Motiv erscheint, enthält, erfasst, eine Position des Messlicht-Bestrahlungsbereichs aus dem Motivbild detektiert und das Messbild auf einem Anzeige anzeigt, wobei die Messmarkierung gemäß der Position des Messlicht-Bestrahlungsbereichs eingestellt ist.

## Revendications

1. Système d'endoscope comprenant :
un dispositif de source de lumière (14) ;
un endoscope (12) configuré pour irradier un sujet avec une lumière de mesure ; et
un dispositif processeur (16) qui inclut un processeur et est configuré pour générer une image de sujet liée au sujet,
dans lequel le processeur est configuré pour effectuer au moins l'une de un premier contrôle qui interdit une commutation d'un mode vers un mode de mesure de longueur dans lequel une image de mesure dans laquelle un marqueur de mesure utilisé pour mesurer une taille du sujet est affiché est affichée sur un affichage dans un cas où une opération pour commuter un mode vers le mode de mesure de longueur est effectuée et une condition de réglage liée au dispositif de source de lumière, à l'endoscope ou au dispositif processeur correspond à une condition de réglage d'interdiction prédéterminée ;
un deuxième contrôle qui désactive une opération de changement de réglage pour la condition de réglage dans un cas où l'opération de changement de réglage est effectuée dans le mode de mesure de longueur et la condition de réglage à laquelle il doit être changée par l'opération de changement de réglage correspond à la condition de réglage d'interdiction ; ou
un troisième contrôle qui commute un mode vers un autre mode à partir du mode de mesure de longueur dans un cas où l'opération de changement de réglage pour la condition de réglage est effectuée dans le mode de mesure de longueur et la condition de réglage à laquelle il doit être changée par l'opération de changement de réglage correspond à la condition de réglage d'interdiction ;
dans lequel l'endoscope (12) inclut une unité d'émission de lumière de mesure (23) qui émet la lumière de mesure et un système optique de capture d'image qui est disposé dans une partie d'extrémité distale à une position différente d'une position de l'unité d'émission de lumière de mesure et reçoit de lumière du sujet, et la lumière de mesure est émise obliquement par rapport à un axe optique du système optique de capture d'image, et
le processeur est configuré pour acquérir l'image de sujet qui inclut une région d'irradiation de lumière de mesure apparaissant sur le sujet en raison d'une irradiation avec la lumière de mesure, détecter une position de la région d'irradiation de lumière de mesure à partir de l'image de sujet, et afficher l'image de mesure sur un affichage, dans lequel le marqueur de mesure est défini en fonction de la position de la région d'irradiation de lumière de mesure.

2. Système d'endoscope selon la revendication 1,
dans lequel la condition de réglage d'interdiction inclut une première condition de réglage d'interdiction qui provoque l'empêchement de la détection d'une position d'une région d'irradiation de lumière de mesure, qui apparaît sur le sujet en raison d'une irradiation avec la lumière de mesure, à partir de l'image de sujet.

3. Système d'endoscope selon la revendication 2,
dans lequel l'image de sujet inclut une image de lumière de mesure basée sur une couleur de la lumière de mesure,
la condition de réglage inclut un mode d'observation spécial dans lequel l'image de lumière de mesure n'est pas utilisée pour l'affichage de l'image de mesure, et la première condition de réglage d'interdiction inclut le mode d'observation spécial.

4. Système d'endoscope selon la revendication 3,
dans lequel le processeur est configuré pour effectuer un contrôle afin d'annuler le mode d'observation spécial et de commuter un mode vers le mode de mesure de longueur au lieu d'interdire une commutation d'un mode vers le mode de mesure de longueur dans un cas où une opération pour commuter un mode vers le mode de mesure de longueur est effectuée dans le mode d'observation spécial.

5. Système d'endoscope selon la revendication 3 ou la revendication 4,
dans lequel l'image de lumière de mesure est une image rouge.

6. Système d'endoscope selon l'une quelconque des revendications 1 à 5,
dans lequel la condition de réglage d'interdiction inclut une deuxième condition de réglage d'interdiction qui provoque l'empêchement de l'affichage d'un marqueur de mesure correspondant à une distance d'observation dans l'image de mesure.

7. Système d'endoscope selon la revendication 6,
dans lequel la deuxième condition de réglage d'interdiction inclut une utilisation d'une fonction de zoom qui agrandit ou réduit le sujet dans l'image de sujet.

8. Système d'endoscope selon l'une quelconque des revendications 1 à 7,
dans lequel le processeur est configuré pour notifier que la commutation d'un mode vers le mode de mesure de longueur est interdite dans un cas où le premier contrôle pour interdire la commutation d'un mode vers le mode de mesure de longueur est effectué.

9. Système d'endoscope selon l'une quelconque des revendications 1 à 7,
dans lequel le processeur est configuré pour notifier que l'opération de changement de réglage est désactivée dans un cas où le deuxième contrôle pour désactiver l'opération de changement de réglage est effectué.

10. Système d'endoscope selon l'une quelconque des revendications 1 à 7,
dans lequel le processeur est configuré pour notifier qu'un mode est commuté vers un autre mode dans un cas où le troisième contrôle pour commuter un mode vers un autre mode à partir du mode de mesure de longueur est effectué.

11. Système d'endoscope selon l'une quelconque des revendications 1 à 10,
dans lequel le marqueur de mesure inclut un premier marqueur de mesure indiquant une taille réelle du sujet ou un deuxième marqueur de mesure constitué d'une ligne d'intersection formée sur le sujet par la lumière de mesure et de graduations formées sur la ligne d'intersection et servant d'indice de la taille du sujet.

12. Procédé d'opération d'un processeur d'un système d'endoscope, le système d'endoscope incluant un dispositif de source de lumière (14), un endoscope (12) qui est configuré pour irradier un sujet avec une lumière de mesure, et un dispositif processeur (16) qui inclut le processeur et est configuré pour générer une image de sujet liée au sujet,
dans lequel le processeur effectue au moins l'une de
un premier contrôle qui interdit une commutation d'un mode vers un mode de mesure de longueur dans lequel une image de mesure dans laquelle un marqueur de mesure utilisé pour mesurer une taille du sujet est affiché est affichée sur un affichage dans un cas où une opération pour commuter un mode vers le mode de mesure de longueur est effectuée et une condition de réglage liée au dispositif de source de lumière, à l'endoscope ou au dispositif processeur correspond à une condition de réglage d'interdiction prédéterminée ;
un deuxième contrôle qui désactive une opération de changement de réglage pour la condition de réglage dans un cas où l'opération de changement de réglage est effectuée dans le mode de mesure de longueur et la condition de réglage à laquelle il doit être changée par l'opération de changement de réglage correspond à la condition de réglage d'interdiction ; ou
un troisième contrôle qui commute un mode vers un autre mode à partir du mode de mesure de longueur dans un cas où l'opération de changement de réglage pour la condition de réglage est effectuée dans le mode de mesure de longueur et la condition de réglage à laquelle il doit être changée par l'opération de changement de réglage correspond à la condition de réglage d'interdiction ;
dans lequel l'endoscope (12) inclut une unité d'émission de lumière de mesure (23) qui émet la lumière de mesure et un système optique de capture d'image qui est disposé dans une partie d'extrémité distale à une position différente d'une position de l'unité d'émission de lumière de mesure et reçoit de lumière du sujet, et la lumière de mesure est émise obliquement par rapport à un axe optique du système optique de capture d'image, et
le processeur acquiert l'image de sujet qui inclut une région d'irradiation de lumière de mesure apparaissant sur le sujet en raison d'une irradiation avec la lumière de mesure, détecte une position de la région d'irradiation de lumière de mesure à partir de l'image de sujet, et affiche l'image de mesure sur un affichage, dans lequel le marqueur de mesure est défini en fonction de la position de la région d'irradiation de lumière de mesure.
